# NEUE EUROPÄISCHE PATENTSCHRIFT

(11) **EP 1 025 253 B2**
(45) Veröffentlichungstag und Bekanntmachung des Hinweises auf die Entscheidung über den Einspruch: **29.07.2009**
(45) Hinweis auf die Patenterteilung: 03.03.2004
(21) Anmeldenummer: 98955483.7
(22) Anmeldetag: 20.10.1998
(51) Int. Cl.: C12N 15/90, C12N 15/65, C12N 15/82

(54) **POSITIV-NEGATIV-SELEKTION BEI DER HOMOLOGEN REKOMBINATION**
POSITIVE-NEGATIVE SELECTION FOR HOMOLOGOUS RECOMBINATION
SELECTION PAR GENE MARQUEUR POSITIF OU NEGATIF LORS D'UNE RECOMBINAISON HOMOLOGUE

(30) Priorität: 20.10.1997 EP 97118175
(43) Veröffentlichungstag der Anmeldung: 09.08.2000
(73) Patentinhaber: Roche Diagnostics GmbH, 68305 Mannheim (DE)
(72) Erfinder: AUER, Johannes, D-82445 Schwalgen (DE); SPRENGER, Raimund, D-82362 Weilheim (DE); HONOLD, Konrad, D-82377 Penzberg (DE)
(74) Vertreter: Weiss, Wolfgang
(86) Internationale Anmeldenummer: PCT/EP1998/006616
(87) Internationale Veröffentlichungsnummer: WO 1999/020780

(56) Entgegenhaltungen:
- EP-A- 0 386 766
- EP-A- 0 455 460
- WO-A-91/06667
- WO-A-92/09631
- WO-A-94/29436
- WO-A-95/06723
- WO-A-97/08186
- WO-A1-93/09222
- US-A- 5 464 764
- US-A- 5 464 764
- MEDIN J A ET AL: "Viral vectors for gene therapy of hematopoietic cells" IMMUNOTECHNOLOGY, Bd. 3, März 1997, Seite 3-19 XP004075435 AMSTERDAM NL
- MORELLE C: "RECOMBINAISON HOMOLOGUE ET CIBLAGE GENIQUE" BIOFUTUR., Nr. 134, 1. Mai 1994, Seiten 1, 3-12, XP000449127 PARIS FR
- DATABASE WPI Section Ch, Week 8939 Derwent Publications Ltd., London, GB; Class B04, AN 89-280998 XP002096031 & JP 01 203975 A (KONICA CORP) , 16. August 1989
- MANSOUR S L ET AL.: 'Disruption of the protooncogene int-2 in mouse embryo-derived stem cells: a general strategy for targeting mutations to non-selectable genes' NATURE Bd. 336, 1988, SALT LAKE CITY, Seiten 348 - 352
- HALLMANN A AND SUMPER M: 'The Chlorella hexose/H+ symporter is a useful selectable marker and biochemical reagent when expressed in Volvox' PROC. NATL. ACAD.SCI. USA Bd. 93, 1996, Seiten 669 - 673
- LE MOUELLIC H ET AL.: 'Homeosis in the Mouse Induced by a Null Mutation in the Hox-3.1 Gene' CELL Bd. 6, 1992, Seiten 251 - 264
- YAGI T ET AL.: 'Homologous recombination at c-fyn locus of mouse embryonic stem cells with use of diphteria toxin A-fragment gene in negative selection' PROC. NATL. ACAD. SCI. USA Bd. 87, 1990, Seiten 9918 - 9922

## Beschreibung

Die Erfindung betrifft ein Verfahren zur Einführung einer fremden DNA in das Genom einer Zielzelle durch homologe Rekombination sowie für die homologe Rekombination geeignete DNA-Konstrukte.

Verfahren zur Einführung fremder DNA in das Genom eukaryontischer Zellen durch homologe Rekombination sind bekannt (siehe z.B. WO 90/11354, WO 91/09955). Dabei wird eine Ausgangszelle mit einem DNA-Konstrukt transfiziert, welches mindestens eine, vorzugsweise zwei DNA-Sequenzabschnitte enthält, die homolog zu Bereichen des Genoms der zur transfizierenden Zelle sind, ein positives Selektionsmarkergen und gegebenenfalls ein negatives Selektionsmarkergen. Weiterhin kann das DNA-Konstrukt eine heterologe Expressionskontrollsequenz enthalten, wenn ein in der transfizierten Zelle normalerweise stummes Gen aktiviert werden soll. Die transfizierten Zellen werden unter Bedingungen kultiviert, bei denen eine Selektion auf das Vorhandensein des positiven Selektionsmarkergens stattfindet, welches bei Expression zu einem selektierbaren Phänotyp führt.

Um Zellen, in denen eine homologe Rekombination stattgefunden hat, von Zellen zu unterscheiden, in denen nur eine zufällige Integration des Vektors in das Genom der Wirtszelle erfolgt ist, zu unterscheiden, erfolgt üblicherweise ein zweiter Selektionsschritt. Hierzu verwendet man ein negatives Selektionsmarkergen, wie etwa das HSV-Thymidinkinasegen (HSV-TK), bei dessen Vorhandensein Zellen in Gegenwart eines Selektionsmittels, z.B. Ganciclovir, zerstört werden. Bei homologer Rekombination verliert die Zelle das HSV-Thymidinkinasegen, so daß Zellen gegenüber Ganciclovir resistent sind. Zellen, in deren Genom der Targetingvektor durch zufällige, nichthomologe Integration eingebaut wurde, verlieren das HSV-TK-Gen nicht und sind deshalb sensitiv gegenüber Ganciclovir. Für diese Art der Selektion durch HSV-TK/Ganciclovir werden vorzugsweise Zellen benutzt, die kein funktionsfähiges Thymidinkinasegen enthalten (z.B. CEM tk von Ogden Bioservices Corp., Rockville MD, USA, Kat. Nr. 491).

Andere für die homologe Rekombination verwendete Wirtzellen besitzen jedoch ein eigenes Thymidinkinasegen. Dieses zelluläre Thymidinkinasegen verursacht aber bei der negativen Selektion Hintergrundprobleme. So kann es beispielsweise beim Screening zum Verlust von homolog rekombinierten Klonen kommen. Ähnliche Probleme treten auch bei anderen negativen Selektionsmarkergenen auf, die für ein Genprodukt kodieren, gegen dessen Expression nach der Transfektion selektioniert werden muß.

Die Verwendung von an der Zelloberfläche lokalisierten Polypeptiden als positiver Transfektionsmarker ist bekannt. So beschreibt z.B. WO 95/06723 ein Verfahren zur Markierung von Zellen unter Verwendung eines partiell deletierten Zelloberflächenrezeptorgens.

Zur Vermeidung der bei den bisher verwendeten negativen Selektionsmarkergenen auftretenden Problem wird erfindungsgemäß ein negatives Selektionsmarkergen verwendet, welches für ein an der Zelloberfläche lokalisiertes Polypeptid kodiert.

Ein Gegenstand der vorliegenden Erfindung ist somit ein Verfahren zur Einführung fremder DNA in eine Wirtszelle durch homologe Rekombination, wobei die Wirtszelle mit einem rekombinanten Vektor transfiziert wird, umfassend zwei flankierende, zu einer Zielsequenz im Genom der Wirtszelle homologe Nukleotidsequenzen, innerhalb derer sich eine für einen positiven Selektionsmarker kodierende Nukleotidsequenz befindet, und außerhalb der flankierenden Sequenzen eine für einen negativen Selektionsmarker kodierende Nukleotidsequenz, die jeweils operativ verknüpft sind mit einer in der Wirtszelle aktiven Expressionskontrollsequenz,
**dadurch gekennzeichnet,**
dass als negatives Selektionsmarkergen mindestens eine für ein an der Zelloberfläche lokalisiertes Polypeptid kodierende Nukleotidsequenz verwendet wird, wobei nach einer Integration des Vektors durch homologe Rekombination in das Genom der Zelle das negative Selektionsmarkergen nicht exprimiert wird und nach einer zufälligen Integration des Vektors in das Genom der Zelle das negative Selektionsmarkergen exprimiert und dessen Genprodukt an der Zelloberfläche präsentiert wird,
wobei man einen Seiektionsschritt auf das Vorhandensein des positiven Selektionsmarkergens und einen weiteren Selektionsschritt auf die Abwesenheit des negativen Selektionsmarkergens durchführt, und die Selektion auf Abwesenheit des negativen Selektionsmarkergens (a) Inkontaktbringen der transfizierten Zellen mit einem Bindemolekül, das an das Genprodukt des negativen Selektionsmarkergens bindet, und (b) Abtrennen der das gebundene Bindemolekül enthaltenden Zellen umfasst.

Erfindungsgemäß wird somit zur Vermeidung eines negativen Selektionsverfahrens, bei dem man mit einem für die Zelle toxischen Selektionsmittel arbeitet, ein für ein oberflächenlokalisiertes Polypeptid kodierendes negatives Selektionsmarkergen an einer entsprechenden Stelle in den Vektor für die homologe Rekombination eingesetzt. Vorzugsweise verwendet man ein negatives Selektionsmarkergen, welches für ein in der Wirtszelle normalerweise nicht vorkommendes Polypeptid kodiert.

Durch das erfindungsgemäße Verfahren treten Probleme mit der Toxizität oder mit Hintergrundsignalen, wie sie im Fall der TK-Selektion beschrieben sind, nicht auf. Ein weiterer Vorteil des erfindungsgemäßen Verfahrens ist, daß die Anzahl an transfizierten Zellen, die auf die Expression des Zielgens hin untersucht werden müssen, deutlich erniedrigt wird.

Die Wirtszelle ist vorzugsweise eine eukaryontische Zelle, besonders bevorzugt eine Säugerzelle und am meisten bevorzugt eine humane Zelle.

Zur Identifizierung und Isolierung von Zellen, in denen eine homologe Rekombination stattgefunden hat, führt man erfindungsgemäß einen Selektionsschritt auf das Vorhandensein des positiven Selektionsmarkergens und einen weiteren Selektionsschritt auf die Abwesenheit des negativen Selektionsmarkergens durch.

Der Selektionsschritt auf das Vorhandensein des positiven Selektionsmarkergens kann auf übliche Weise erfolgen. Als positives Selektionsmarkergen kann man dabei ein beliebiges, insbesondere für eukaryontische Zellen geeignetes Selektionsmarkergen verwenden, welches bei Expression zu einem selektierbaren Phänotyp führt, z.B. Antibiotikumresistenz, oder Auxotrophie. Vorzugsweise werden Antibiotikumresistenzgene verwendet, z.B. das Neomycin-, Kanamycin-, Geneticin-oderHygromycin-Resistenzgen. Ein besonders bevorzugtes positives Selektionsmarkergen ist das Neomycinphosphotransferasegen.

Das für das erfindungsgemäße Verfahren verwendete negative Selektionsmarkergen kodiert für ein Genprodukt, welches an der Oberfläche der Wirtszelle präsentiert wird, vorzugsweise für ein membranständiges Polypeptid. Bevorzugte Beispiele für solche membranständigen Polypeptide sind etwa der LNGF-, der CD24-, der LDL- oder der trk-Rezeptor oder ein membranständiges, die Ligandenbindedomäne des jeweiligen Rezeptors enthaltendes Rezeptorfragment. Geeignete Rezeptorfragmente, bei denen die intrazelluläre Domäne vollständig oder teilweise deletiert ist oder auf solche Weise modifiziert ist, daß der an der Oberfläche präsentierte Rezeptor keine Signaltransduktion bewirken kann, sind in WO 95/06723 beschrieben. Ein besonders bevorzugtes Beispiel für ein solches Rezeptorfragment ist eine Deletionsmutante des LNGF-Rezeptors (dLNGFR), bei der es sich um ein Fragment des humanen niedrigaffinen Rezeptors des Nervenwachstumsfaktors handelt, dessen intrazelluläre und signaltransduzierende Domäne deletiert wurde (WO 95/06723).

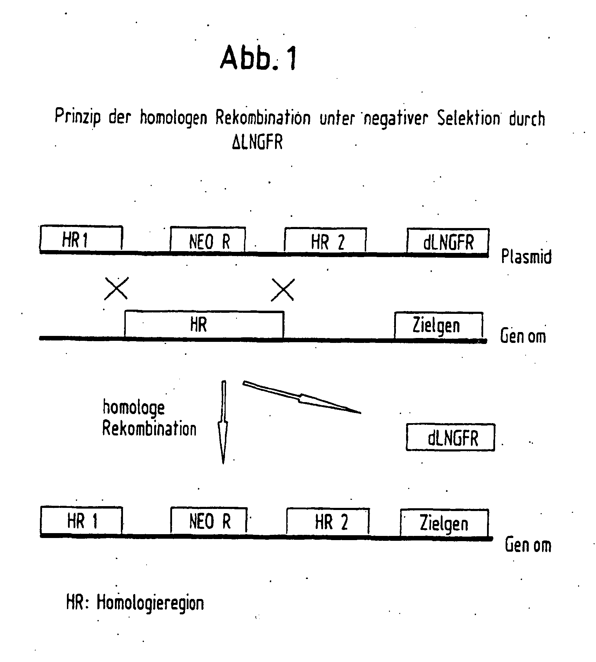

In Abbildung 1 ist schematisch das Prinzip der homologen Rekombination unter negativer Selektion durch dLNGFR gezeigt. Dieses Selektionsprinzip kann selbstverständlich auch auf andere, für oberflächenassoziierte Polypeptide kodierende Selektionsmarkergene übertragen werden. Als rekombinanter Vektor wird ein Plasmid verwendet, welches zwei flankierende zur gewünschten Zielsequenz homologe Nukleinsäureabschnitte (HR1, HR2) und dazwischen das positive Selektionsmarkergen das Neomycinresistenzgen (NeoR) enthält. Außerhalb der beiden flankierenden homologen Nukleotidsequenzen ist auf dem Plasmid eine für dLNGFR kodierende Nukleotidsequenz angeordnet.

Bei einer homologen Rekombination mit einer Region im Bereich des Zielgens (HR) erfolgt eine Integration der Regionen HR1, NeoR und HR2 in das Genom. Die für dLNGFR kodierende Sequenz wird hingegen nicht in das Genom integriert. Im Unterschied dazu bleibt bei einer zufälligen Integration des Plasmids im Genom der Wirtszelle das dLNGFR-Gen in expressionsfähiger Form erhalten.

Die erfindungsgemäße Selektion auf die Abwesenheit des negativen Selektionsmarkergens in der transfizierten Wirtszelle umfasst die Schritte:
(a) Inkontaktbringen der transfizierten Zelle mit einem Bindemolekül, das an das Genprodukt des negativen Selektionsmarkergens bindet, und
(b) Abtrennen der das gebundene Bindemolekül enthaltenden Zellen.

Als Bindemoleküle werden Substanzen verwendet, die eine spezifische und vorzugsweise hochaffine Bindung mit dem negativen Selektionsmarker eingehen können. Vorzugsweise werden solche Bindemoleküle verwendet, die keine störende Kreuzreaktivität mit anderen Oberflächenkomponenten der Wirtszelle aufweisen. Beispiele für Bindemoleküle sind Antikörper, z.B. polyklonale oder monoklonale Antikörper, Antikörperfragmente etc., die gegen das Genprodukt des negativen Selektionsmarkergens gerichtet sind. Geeignete Antikörper gegen dLNGFR sind beispielsweise aus WO 95/06723 bekannt. Bei Verwendung eines Rezeptors als negativen Selektionsmarker kann als Bindemolekül selbstverständlich auch ein natürlicher Bindepartner des Rezeptors, z.B. der Rezeptorligand, oder ein Analogon davon verwendet werden. Ein Beispiel für einen solchen Rezeptorliganden sind NGF als Ligand von LNGFR.

Um die Abtrennung der mit dem negativen Selektionsmarker markierten Zellen zu erleichtern, kann man ein Bindemolekül verwenden, welches an eine Festphase gekoppelt ist, wobei diese Koppelung durch Adsorption, kovalente Bindung oder über ein hochaffines Bindepaar (z.B. Streptavidin/-Biotin) erfolgen kann. Die Art der Festphase ist für das erfindungsgemäße Verfahren im allgemeinen nicht kritisch, vorzugsweise werden solche Festphasen verwendet, die eine leichte Abtrennung der den negativen Selektionsmarker präsentierenden Zellen von unmarkierten Zellen ermöglichen. Die Festphase kann daher beispielsweise in Form einer Chromatographiesäule vorliegen, besonders bevorzugt sind jedoch partikuläre Festphasen wie etwa Microbeads, insbesondere magnetische Microbeads, die eine besonders einfache Abtrennung erlauben.

Alternativ können die transfizierten Zellen auch mit freien Bindemolekülen in Kontakt gebracht werden. In diesem Fall tragen die freien Bindemoleküle vorzugsweise eine Markierungs- oder/und eine Festphasenbindegruppe. Beispiele fürgeeignete Markierungs-oder/und Festphasenbindegruppen sind Biotin, Biotinderivate, z.B. Iminobiotin, Aminobiotin oder Desthiobiotin, Haptene, z.B. Digoxigenin, Fluorescein, Enzyme, z.B. Peroxidase oder alkalische Phosphatase oder Farbstoffe, z.B. Fluoreszenzfarbstoffe wie etwa Fluorescein, Phycoerythrin, Rhodamin, Peridinin-Chlorophyll-Protein, Texasrot oder Derivate davon.

Bei Verwendung eines Bindemoleküls, welches eine Festphasenbindegruppe wie etwa Biotin, ein Biotinderivat oder ein Hapten trägt, kann die mit dem Bindemolekül markierte Zelle an eine Festphase gekoppelt werden, welche mit der Festphasenbindegruppe des Bindemoleküls reagieren kann. Bei Verwendung eines Bindemoleküls, welches eine Biotingruppe trägt, kann man beispielsweise die den negativen Selektionsmarker exprimierenden Zellen durch Bindung an eine Avidin- oder Streptavidin-beschichtete Festphase identifizieren und von unmarkierten Zellen abtrennen.

Bei Verwendung eines Bindemoleküls, welches eine enzymatische Markierungsgruppe trägt, können die den negativen Selektionsmarker exprimierenden Zellen nach Zugabe eines Enzymsubstrats durch eine enzymkatalysierte Farbreaktion identifiziert und gegebenenfalls von unmarkierten Zellen abgetrennt werden. Diese Identifizierung kann beispielsweise durch Auftragen der Zellen auf einem Objektträger und anschließende mikroskopische Analyse erfolgen.

Wenn man ein Bindemolekül verwendet, welches einen Fluoreszenzfarbstoff trägt, können die den negativen Selektionsmarker exprimierenden Zellen durch durchflußzytometrische Analyse identifiziert und von unmarkierten Zellen abgetrennt werden. Dieses Abtrennverfahren ist schnell und einfach und kann in üblichen FACS Geräten durchgeführt werden, welche das Setzen von Fluoreszenz-Fenstern und eine Zellsortierung ermöglichen.

Ein weiterer Gegenstand der vorliegenden Erfindung ist ein rekombinanter Vektor, für die homologe Rekombination in Säugerzellen der zur Verwendung als Transfektionsvektor im erfindungsgemäßen Verfahren geeignet ist. Dieser Vektor umfaßt:
(a) zwei flankierende, zu einer Zielsequenz in einer Zelle homologe Nukleotidsequenzen,
(b) eine für einen positiven Selektionsmarker kodierende Nukleotidsequenz unter Kontrolle einer in der Zelle aktiven Expressionskontrollsequenz, die sich innerhalb der zwei flankierenden Sequenzen gemäß (a) befindet,
(c) eine für einen negativen Selektionsmarker kodierende Nukleotidsequenz unter Kontrolle einer in der Zelle aktiven Expressionskontrollsequenz, die sich außerhalb der flankierenden homologen Nukleotidsequenzen befindet und deren Expressionsprodukt ein an der Zelloberfläche lokalisiertes Polypeptid ist.

Wenn der rekombinante Vektor zur Aktivierung eines in der Wirtszelle endogen vorliegenden Gens eingesetzt werden soll, enthält er zwischen den zwei flankierenden homologen Nukleotidsequenzen noch eine heterologe Expressionskontrollsequenz, die in der Wirtszelle aktiv ist. Diese Expressionskontrollsequenz umfasst einen Promotor und vorzugsweise weitere expressionsverbessernde Sequenzen, z.B. einen Enhancer. Der Promotor kann ein regulierbarer oder ein konsitutiver Promotor sein. Vorzugsweise ist der Promotor ein starker viraler Promotor, z.B. ein SV40- oder ein CMV-Promotor. Besonders bevorzugt ist der CMV-Promotor/Enhancer.

Wenn eine Amplifikation des Zielgens in der transfizierten Wirtzelle gewünscht wird, enthält der rekombinanter Vektor ein Amplifikationsgen zwischen den beiden flankierenden Sequenzen. Beispiele für geeignete Amplifikationsgene sind Dihydrofolatreduktase, Adenosindeaminase, Ornithindecarboxylase etc. Ein besonders bevorzugtes Amplifikationsgen ist das Dihydrofolatreduktasegen, insbesondere ein für eine Dihydrofolatreduktase-Arginin-Mutante kodierendes Gen, die eine geringere Sensitivität für das selektive Agens (Methotrexat) besitzt als das Wildtyppolypeptid (Simonsen et al., Proc. Natl. Acad. Sci. USA 80 (1983), 2495).

Die für den negativen Selektionsmarker kodierende Nukleotidsequenz kann - wie zuvor erläutert - vorzugsweise aus membranständigen Rezeptoren oder membranständigen, die Ligandenbindedomäne des jeweiligen Rezeptors enthaltenden Rezeptorfragmenten ausgewählt werden.

Die flankierenden, zu einer Zielsequenz homologen Nukleotidsequenzen können aus beliebigen chromosomalen Bereichen des Genoms der zu transfizierenden Zelle, die vorzugsweise eine eukaryontische Zelle, besonders bevorzugt eine Säugerzelle und am meisten bevorzugt eine humane Zelle ist, ausgewählt werden. Bei humanen Zellen werden die flankierenden homologen Nukleotidsequenzen vorzugsweise aus dem Bereich der Gene für humane Faktoren, z.B. EPO, tPA, G-CSF, GM-CSF, TPO, Interleukinen, Interferonen, Wachstumsfaktoren, Insulin, insulinartigen Wachstumsfaktor etc. stammen.

Die flankierenden homologen Nukleotidsequenzen können den kodierenden Bereich des Zielgens oder einen Teil davon umfassen. In diesem Teil können sie so ausgewählt werden, daß sie bei homologer Rekombination eine Mutation im kodierenden Bereich des reifen Zielpolypeptids gegenüber der endogen in der Zelle vorliegenden Sequenz verursachen. Diese Mutation kann Substitutionen, Deletionen und Insertionen einzelner Aminosäuren oder ganzer Aminosäureabschnitte umfassen.

Offenbart wird weiterhin die Verwendung von membranständigen Oberflächenrezeptoren als negativer Selektionsmarker in einem Verfahren der homologen Rekombination.

Weiterhin wird die Erfindung durch die nachfolgenden Beispiele und Abbildungen erläutert. Es zeigen:
- Abbildung 1:: Eine schematische Darstellung des Prinzips der homologen Rekombination unter erfindungsgemäßer Verwendung einer negativen Selektion durch dLNGFR,
- Abbildung 2:: die Restriktionskarte des Plasmids pSV-dLNGFR,
- Abb.3a und b:: Ergebnisse einer FACS-Analyse von dLNGFR exprimierenden und nicht exprimierenden Zellen,
- Abbildung 4:: die Restriktionskarte des Plasmids p 187-dLNGFR,
- Abbildung 5:: das Ergebnis einer FACS-Analyse zur Unterscheidung von dLNGFR negativen und positiven Zellen,

### Beispiele

### Methoden

### Rekombinante DNA-Technik

Zur Manipulation von DNA wurden Standardmethoden benutzt, wie sie bei Sambrook, J. et al. (1989) in: Molecular Cloning: A Laboratory Manual, Cold Spring Harbor Laboratory Press, Cold Spring Harbor, New York, beschrieben sind. Die verwendeten molekularbiologischen Reagenzien wurden nach den Angaben des Herstellers eingesetzt.

### Transfektion von humanen Zellinien, Kultivierung und Klonierung

Der Vektor lag gelöst in einer Konzentration von 1 µg/µl bidestilliertem Wasser vor. Um eine hohe Transfektionseffizienz zu gewährleisten, wurden die Zellen mit Hilfe der Elektroporation (BioRad, Genepulser™) unter zuvor als optimal bestimmten Bedingungen transfiziert (960 µF/260 MV/18-22 µS). Als eine geeignete Zellinie wurde die adhärent wachsende humane Fibrosarkomlinie HT1080 (ATCC CCL 121) in einer Konzentration von 10⁷ Zellen/0,8 ml eingesetzt. Vor und nach der Transfektion wurden die Zellen für ca. 10 min auf Eis gehalten, um die Zellmembran wieder zu rekonstituieren.

Transfektierte Zellen wurden in T-175 Kulturflaschen ausgesät und bei 37°C und 7% CO₂ im Brutschrank kultiviert. Nach 24 h wurde Selektionsdruck durch Zugabe von G418 (0,8 µg/ml) angelegt.

Nach 14 Tagen in Kultur zeigten sich resistente Klone in der Kulturschale. Nachdem größere Foci ausgewachsen waren, wurden die Zellen mit PBS gewaschen, trypsiniert und als Einzelzellsuspension gefärbt.

### FACS-Analyse

Die Färbeschritte wurden mit 10⁵ Zellen/Ansatz auf Eis durchgeführt. Der als Primärantikörper zugegebene Anti-dLNGFR-Antikörper aus der Maus wurde durch Zugabe eines Sekundär-Antikörpers aus der Ziege detektiert (α-mIgG-FITC, 1:25, Caltag). Als Kontrolle für unspezifische Bindung wurden die Zellen mit dem Sekundär-Antikörper allein angefärbt. Tote Zellen wurden durch Zugabe von Propidiumiodid (10 µg/ml) detektiert. Die Analysen wurden auf einem FACS-Vantage (Fa. Becton Dickinson) nach den Angaben des Herstellers durchgeführt. Die spezifische Fluoreszenz der dLNGFR-exprimierenden Zellen wurde im FL-1 Kanal, die toten Zellen im FL-3 Kanal erfaßt.

### Beispiel 1

### Herstellung des Expressionskonstrukts für dLNGFR

Das Gen für dLNGFR (WO 95/06723, Boehringer Mannheim GmbH), das 965 Bp umfaßt, wurde mit Hilfe der PCR-Technik amplifiziert. Durch die verwendeten Primer wurden an beiden Enden Schnittstellen für die Enzyme EcoRI bzw. Sall eingeführt. Nach der Amplifikation wurden die PCR-Fragmente mit beiden Enzymen geschnitten.

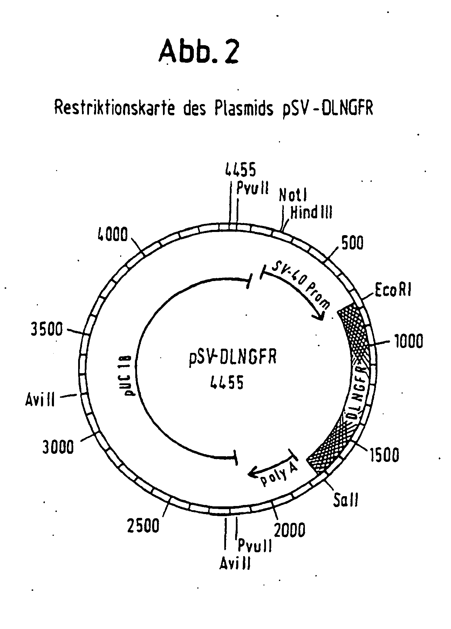

Der Vektor pSV1, der den frühen SV40 Promotor und das SV40 polyA-Signal enthält (Okayama und Berg, Mol. Cell. Biol. 3 (1983), 280-289; Mulligan und Berg, Proc. Natl, Acad. Sci. USA 78 (1981), 2072-2076) wurde ebenfalls mit EcoRI und Sall geschnitten. Der isolierte Vektor besitzt eine Größe von 3490 Bp. Das dLNGFR-Fragment wurde in den Vektor pSV1 ligiert. Das Gen für dLNGFR steht unter der Expressionskontrolle des frühen SV40 Promotors und des SV40 Poly-Signals. Die gesamte Expressionskassette umfaßt 1900 Bp. Der resultierende Vektor pSV-DLNGR ist in Abb. 2 dargestellt.

### Beispiel 2

### Test der Expressionskassette auf Funktionalität

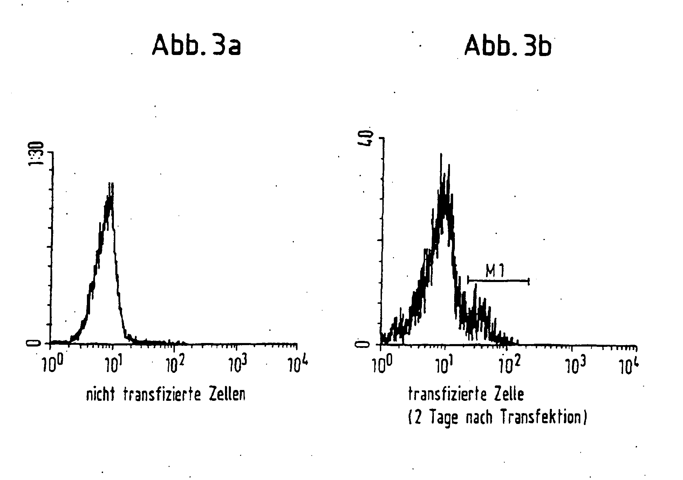

Zellen der Linie HT1080 wurden mit dem Plasmid pSV-DLNGFR wie oben beschrieben transient transfiziert. Nach zwei Tagen Wachstum wurden die Zellen auf Expression von dLNGFR mit Hilfe des monoklonalen Anti-dLNGFR-Antikörpers analysiert. Das Ergebnis ist in Abbildung 3 wiedergegeben, die zeigt, daß dLNGFR-exprimierende und nicht-exprimierende Zellen durch FACS-Analyse unterscheidbar sind. Sie zeigt weiter, daß die Reaktion des Anti-dLNGFR-Antikörpers spezifisch ist für transfizierte Zellen.

### Beispiel 3

### Klonierung des dLNGFR-Expressionskassette in einen Gentargetingvektor

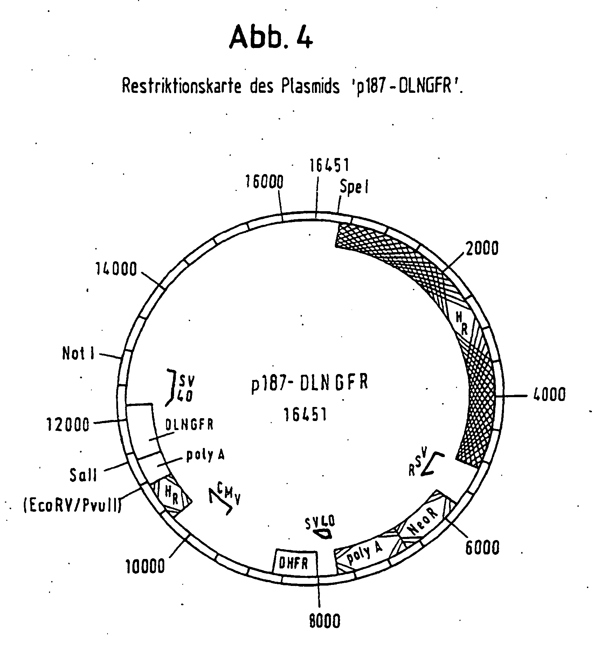

Die dLNGFR-Expressionskassette wurde mit den Restriktionsenzymen Notl und Pvull aus pSV-DLNGFR isoliert. Der Targetingvektor 'p187' für das humane EPO-Gen (beschrieben in EP 97 112 649.5 und EP 97 112 640.5 siehe Abb. 4b) wurde mit NotI und EcoRV geschnitten. Das 14551 Bp große Vektorfragment wurde isoliert und mit der dLNGFR-Expressionskassette ligiert (Abb.4) Das resultierende Plasmid 'p187-DLNGFR' wurde in E.coli transferiert und darin propagiert.

### Beispiel 4

### Test auf negative Selektion im FACS-Scan

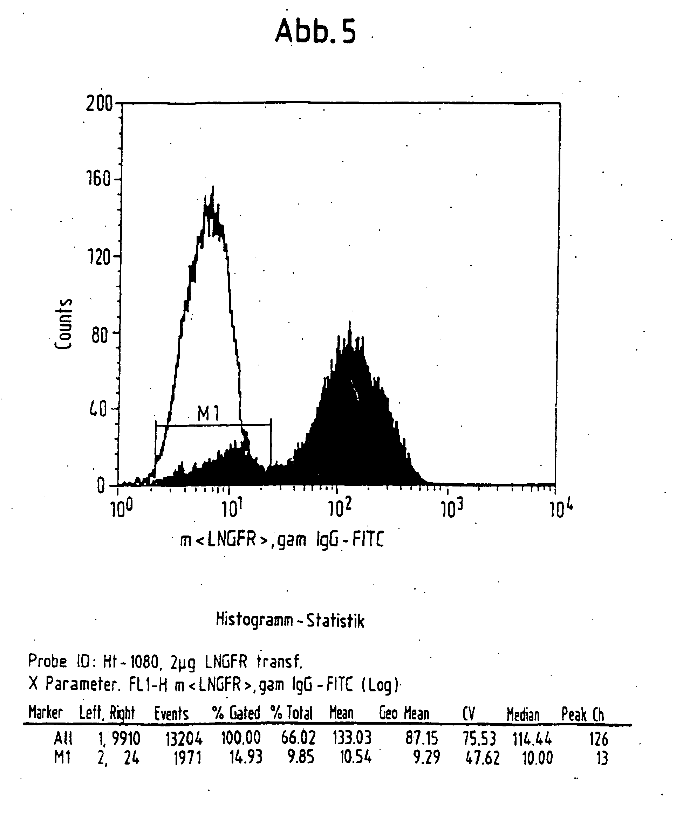

HT1080-Zellen wurden mit p187-DLNGFR transfiziert und auf stabile Integration selektiert, d.h. 24 Stunden nach der Transfektion wurde G418 zum Medium zugegeben. Die erste FACS-Analyse wurde nach ca. 3 Wochen Wachstum durchgeführt und zwar nach der Bildung erster Foci, deren Zellen gepoolt wurden. Wie in Abbildung 5 gezeigt, können nach dieser Zeit dLNGFR-negative Zellen, hier 14% der Population, von den dLNGFR-exprimierenden Zellen durch FACS-Analyseunterschieden werden.

In dieser Zellpopulation befinden sich neben dem selten auftretenden Ereignis der homologen Rekombination auch solche Zellen, die eine zu geringe Rezeptordichte auf ihrer Oberfläche aufweisen und daher vom Nachweissystem nicht erkannt werden. Auf diese Weise kann jedoch die Anzahl der Klone, die anschließend auf Expression des Zielgens getestet werden müssen, deutlich reduziert werden (hier 14 von 100%).

Befindet sich in einem Transfektionsansatz kein Klon, der den Targetingvektor homolog rekombiniert vorliegen hat, so kann dieser Umstand im Vergleich zum konventionellen Screening ohne großen Arbeitsaufwand gezeigt werden. Das Fehlen homolog rekombinierter Klone wird durch das Auftreten einer zu 100% mit Anti-dLNGFR-Antikörper reagierenden Population demonstriert. Das weitere Screening auf Expression des Zielgens kann dann entfallen.

## Patentansprüche

1. Verfahren zur Einführung fremder DNA in eine Wirtszelle durch homologe Rekombination, wobei die Wirtszelle mit einem rekombinanten Vektor transfiziert wird, umfassend zwei flankierende, zu einer Zielsequenz im Genom der Wirtszelle homologe Nukleotidsequenzen, innerhalb derer sich eine für einen positiven Selektionsmarker kodierende Nukleotidsequenz befindet, und außerhalb der flankierenden Sequenzen eine für einen negativen Selektionsmarker kodierende Nukleotidsequenz, die jeweils operativ verknüpft sind mit einer in der Wirtszelle aktiven Expressionskontrollsequenz,
**dadurch gekennzeichnet,**
**dass** als negatives Selektionsmarkergen mindestens eine für ein an der Zelloberfläche lokalisiertes Polypeptid kodierende Nukleotidsequenz verwendet wird, wobei nach einer Integration des Vektors durch homologe Rekombination in das Genom der Zelle das negative Selektionsmarkergen nicht exprimiert wird und nach einer zufälligen Integration des Vektors in das Genom der Zelle das negative Selektionsmarkergen exprimiert und dessen Genprodukt an der Zelloberfläche präsentiert wird,
wobei man einen Selektionsschritt auf das Vorhandensein des positiven Selektionsmarkergens und einen weiteren Selektionsschritt auf die Abwesenheit des negativen Selektionsmarkergens durchführt, und die Selektion auf Abwesenheit des negativen Selektionsmarkergens (a) Inkontaktbringen der transfizierten Zellen mit einem Bindemolekül, das an das Genprodukt des negativen Selektionsmarkergens bindet, und (b) Abtrennen der das gebundene Bindemolekül enthaltenden Zellen umfasst.

2. Verfahren nach Anspruch 1,
**dadurch gekennzeichnet,**
**dass** ein negatives Selektionsmarkergen verwendet wird, das für einen LNGF-, einen CD24-, einen LDL-, einen trk-Rezeptor oder ein membranständiges, die Ligandenbindedomäne enthaltendes Fragment des Rezeptors kodiert.

3. Verfahren nach Anspruch 1 oder 2,
**dadurch gekennzeichnet,**
**dass** als Bindemolekül ein Antikörper verwendet wird, der gegen das Genprodukt des negativen Selektionsmarkergens gerichtet ist.

4. Verfahren nach Anspruch 1 oder 2,
**dadurch gekennzeichnet,**
**dass** als Bindemolekül ein natürlicher Bindepartner des negativen Selektionsmarkergens oder ein Analogon davon verwendet wird.

5. Verfahren nach einem der Ansprüche 1 bis 4,
**dadurch gekennzeichnet,**
**dass** man ein Bindemolekül verwendet, das an eine Festphase gekoppelt ist.

6. Verfahren nach Anspruch 5,
**dadurch gekennzeichnet,**
**dass** man als Festphase magnetische Microbeads verwendet.

7. Verfahren nach einem der Ansprüche 1 bis 6,
**dadurch gekennzeichnet,**
**dass** man ein Bindemolekül verwendet, das eine Markierungs-oder/und Festphasebindegruppe trägt.

8. Verfahren nach Anspruch 7,
**dadurch gekennzeichnet,**
**dass** die Markierungs- oder/und Festphasebindegruppe ausgewählt wird aus Biotin, Biotinderivaten, Haptenen, Enzymen und Farbstoffen.

9. Verfahren nach Anspruch 8,
**dadurch gekennzeichnet,**
**dass** man Biotin oder ein Biotinderivat ausgewählt aus Iminobiotin, Aminobiotin und Desthiobiotin verwendet.

10. Verfahren nach Anspruch 8,
**dadurch gekennzeichnet,**
**dass** man als Enzym eine alkalische Phosphatase oder Peroxidase verwendet.

11. Verfahren nach Anspruch 8,
**dadurch gekennzeichnet,**
**dass** man als Farbstoff einen Fluoreszenzfarbstoff verwendet.

12. Verfahren nach Anspruch 11,
**dadurch gekennzeichnet,**
**dass** man als Fluoreszenzfarbstoff Fluorescein, Phycoerythrin, Rhodamin, Peridinin-Chlorophyll-Protein oder Texasrot verwendet.

13. Verfahren nach Anspruch 9,
**dadurch gekennzeichnet,**
**dass** die den negativen Selektionsmarker exprimierenden Zellen durch Bindung an eine Avidin- oder Streptavidin-beschichtete Festphase identifiziert werden.

14. Verfahren nach Anspruch 10,
**dadurch gekennzeichnet,**
**dass** die den negativen Selektionsmarker exprimierenden Zellen durch eine enzymkatalytische Farbreaktion identifiziert werden.

15. Verfahren nach einem der Ansprüche 11 oder 12,
**dadurch gekennzeichnet,**
**dass** die den negativen Selektionsmarker exprimierenden Zellen durch durchflusszytometrische Analyse identifiziert werden.

16. Verfahren nach einem der vorhergehenden Ansprüche,
**dadurch gekennzeichnet,**
**dass** die Zelle eine eukaryontische Zelle, vorzugsweise eine Säugerzelle und besonders bevorzugt eine humane Zelle ist.

17. Rekombinanter Vektor für die homologe Rekombination in Säugerzellen, umfassend
(a) zwei flankierende, zu einer Zielsequenz in einer Zelle homologe Nukleotidsequenzen,
(b) eine für einen positiven Selektionsmarker kodierende Nukleotidsequenz unter Kontrolle einer in der Zelle aktiven Expressionskontrollsequenz, die sich innerhalb der flankierenden Sequenzen gemäß (a) befindet, und
(c) eine für einen negativen Selektionsmarker kodierende Nukleotidsequenz unter Kontrolle einer in der Zelle aktiven Expressionskontrollsequenz, die sich außerhalb der flankierenden homologen Nukleotidsequenzen befindet und deren Expressionsprodukt ein an der Zelloberfläche lokalisiertes Polypeptid ist.

18. Vektor nach Anspruch 17,
**dadurch gekennzeichnet,**
**dass** die flankierenden homologen Nukleotidsequenzen ausgewählt sind aus dem Bereich eines Gens für EPO, tPA, G-CSF, GM-CSF, TPO, ein Interleukin, ein Interferon, einen Wachstumsfaktor, Insulin oder insulinartigen Wachstumsfaktor.

19. Vektor, nach einem der Ansprüche 17 oder 18,
**dadurch gekennzeichnet,**
**dass** die für den positiven Selektionsmarker kodierende Nukleotidsequenz ein Neomycin-, Kanamycin-, Geneticin-, oder Hygromycin-Resistenzgen ist.

20. Vektor nach einem der Ansprüche 17 bis 19,
**dadurch gekennzeichnet,**
**dass** die für den negativen Selektionsmarker kodierende Nukleotidsequenz eine für einen LNGF-, CD24-, LDL-, trk-Rezeptor oder ein membranständiges, die Ligandenbindedomäne davon enthaltendes Fragment kodierende Sequenz ist.

21. Vektor nach einem der Ansprüche 17 bis 20,
**dadurch gekennzeichnet,**
**dass** er innerhalb der flankierenden Sequenzen weiterhin eine heterologe Expressionskontrollsequenz enthält.

22. Vektor nach Anspruch 21,
**dadurch gekennzeichnet,**
**dass** die Expressionskontrollsequenz einen CMV-Promotor umfasst.

23. Vektor nach einem der Ansprüche 17 bis 22,
**dadurch gekennzeichnet,**
**dass** er innerhalb der flankierenden Sequenzen weiterhin ein Amplifikationsgen enthält.

24. Vektor nach einem der Ansprüche 17 bis 23,
**dadurch gekennzeichnet,**
**dass** die flankierenden homologen Nukleotidsequenzen den kodierenden Bereich des Zielgens oder einen Teil davon umfassen.

25. Vektor nach Anspruch 24
**dadurch gekennzeichnet,**
**dass** die flankierenden homologen Nukleotidsequenzen so ausgewählt sind, dass bei homologer Rekombination eine Mutation im kodierenden Bereich des reifen Zielpolypeptids auftritt.

26. Verwendung eines Vektors nach einem der Ansprüche 17 bis 25 in einem Verfahren der homologen Rekombination.

## Claims

1. Method for introducing foreign DNA into a host cell by homologous recombination in which the host cell is transfected with a recombinant vector comprising two flanking nucleotide sequences which are homologous to a target sequence in the genome of the host cell and inside of which a nucleotide sequence coding for a positive selection marker is located, and a nucleotide sequence outside the flanking sequences which codes for a negative selection marker, which are each operatively linked to an expression control sequence which is active in the host cell,
**characterized in that**
at least one nucleotide sequence coding for a polypeptide located on the cell surface is used as the negative selection marker gene wherein after integration of the vector into the genome of the cell by homologous recombination the negative selection marker gene is not expressed and after a random integration of the vector into the genome of the cell the negative selection marker gene is expressed and its gene product is presented on the cell surface, wherein a selection step for the presence of the positive selection marker gene is carried out and a further selection step for the absence of the negative selection marker gene is carried out, and the selection for the absence of the negative selection marker gene comprises (a) contacting the transfected cells with a binding molecule which binds to the gene product of the negative selection marker gene and (b) separating the cells containing the bound binding molecule.

2. Method according to claim 1,
**characterized in that**
a negative selection marker gene is used which codes for an LNGF receptor, a CD24 receptor, an LDL receptor, a trk receptor or a membrane-based fragment of the receptor containing the ligand binding domain.

3. Method according to claim 1 or 2,
**characterized in that**
an antibody is used as the binding molecule which is directed against the gene product of the negative selection marker gene.

4. Method according to claim 1 or 2,
**characterized in that**
a natural binding partner of the negative selection marker gene or an analogue thereof is used as the binding molecule.

5. Method according to one of the claims 1 to 4,
**characterized in that**
a binding molecule is used which is coupled to a solid phase.

6. Method according to claim 5,
**characterized in that**
magnetic microbeads are used as a solid phase.

7. Method according to one of the claims 1 to 6,
**characterized in that**
a binding molecule is used that carries a labelling group or/and a solid phase binding group.

8. Method according to claim 7,
**characterized in that**
the labelling group or/and solid phase binding group is selected from biotin, biotin derivatives, haptens, enzymes and dyes.

9. Method according to claim 8,
**characterized in that**
biotin or a biotin derivative selected from iminobiotin, aminobiotin and desthiobiotin is used.

10. Method according to claim 8,
**characterized in that**
an alkaline phosphatase or peroxidase is used as the enzyme.

11. Method according to claim 8,
**characterized in that**
a fluorescent dye is used as the dye.

12. Method according to claim 11,
**characterized in that**
fluorescein, phycoerythrin, rhodamine, peridinine-chlorophyl protein or Texas red is used as the fluorescent dye.

13. Method according to claim 9,
**characterized in that**
the cells expressing the negative selection marker are identified by binding to an avidin-coated or streptavidin-coated solid phase.

14. Method according to claim 10,
**characterized in that**
the cells expressing the negative selection marker are identified by an enzyme-catalysed colour reaction.

15. Method according to one of the claims 11 or 12,
**characterized in that**
the cells expressing the negative selection marker are identified by flow-cytometric analysis.

16. Method according to one of the previous claims,
**characterized in that**
the cell is a eukaryotic cell, preferably a mammalian cell and particularly preferably a human cell.

17. Recombinant vector for homologous recombination in mammalian cells comprising,
(a) two flanking nucleotide sequences that are homologous to a target sequence in a cell,
(b) a nucleotide sequence coding for a positive selection marker under the control of an expression control sequence that is active in the cell, which is located inside the flanking sequences according to (a) and
(c) a nucleotide sequence coding for a negative selection marker under the control of an expression control sequence that is active in the cell, which is located outside the flanking homologous nucleotide sequences and whose expression product is a polypeptide located on the cell surface.

18. Vector according to claim 17,
**characterized in that**
the flanking homologous nucleotide sequences are selected from the region of a gene for EPO, tPA, G-CSF, GM-CSF, TPO, an interleukin, an interferon, a growth factor, insulin or insulin-like growth factor.

19. Vector according to one of the claims 17 or 18,
**characterized in that**
the nucleotide sequence coding for the positive selection marker is a neomycin, kanamycin, geneticin or hygromycin resistance gene.

20. Vector according to one of the claims 17 to 19,
**characterized in that**
the nucleotide sequence coding for the negative selection marker is a sequence coding for an LNGF receptor, a CD24 receptor, an LDL receptor, a trk receptor or a membrane-based fragment containing the ligand binding domain thereof.

21. Vector according to one of the claims 17 to 20,
**characterized in that** it additionally contains a heterologous expression control sequence inside the flanking sequences.

22. Vector according to claim 21,
**characterized in that**
the expression control sequence contains a CMV promoter.

23. Vector according to one of the claims 17 to 23,
**characterized in that**
it additionally contains an amplification gene inside the flanking sequences.

24. Vector according to one of the claims 17 to 23,
**characterized in that**
the flanking homologous nucleotide sequences contain the coding region of the target gene or a part thereof.

25. Vector according to claim 24,
**characterized in that**
the flanking homologous nucleotide sequences are selected so that a mutation in the coding region of the mature target polypeptide occurs in homologous recombination.

26. Use of a vector according to one of the claims 17 to 25 in a method for homologous recombination.

## Revendications

1. Procédé pour introduire un ADN étranger dans une cellule hôte par recombinaison homologue, où la cellule hôte est transfectée avec un vecteur recombiné comprenant deux séquences nucléotidiques flanquantes, homologues d'une séquence cible dans le génome de la cellule hôte, à l'intérieur desquelles se trouve une séquence nucléotidique codant un marqueur de sélection positif, et à l'extérieur des séquences flanquantes une séquence nucléotidique codant un marqueur de sélection négatif, qui sont liées chacune de manière fonctionnelle avec une séquence de contrôle de l'expression active dans la cellule hôte, **caractérisé en ce qu'**au moins une séquence nucléotidique codant un polypeptide localisé sur la surface cellulaire est utilisée comme gène marqueur de sélection négatif, où après une intégration du vecteur par recombinaison homologue dans le génome de la cellule, le gène marqueur de sélection négatif n'est pas exprimé et après une intégration fortuite du vecteur dans le génome de la cellule, le gène marqueur de sélection négatif est exprimé et son produit génique est présenté sur la surface cellulaire, où on réalise une étape de sélection concernant la présence du gène marqueur de sélection positif et une autre étape de sélection concernant l'absence du gène marqueur de sélection négatif, et la sélection concernant l'absence du gène marqueur de sélection négatif comprend (a) la mise en contact des cellules transfectées avec une molécule de liaison qui se lie au produit génique du gène marqueur de sélection négatif, et (b) la séparation des cellules contenant la molécule de liaison liée.

2. Procédé selon la revendication 1 **caractérisé en ce que** l'on utilise un gène marqueur de sélection négatif qui code un récepteur de LNGF, CD24, LDL, trk ou un fragment membranaire du récepteur contenant le domaine de liaison de ligand.

3. Procédé selon la revendication 1 ou 2 **caractérisé en ce que** l'on utilise comme molécule de liaison un anticorps qui est dirigé contre le produit génique du gène marqueur de sélection négatif.

4. Procédé selon la revendication 1 ou 2 **caractérisé en ce que** l'on utilise comme molécule de liaison un partenaire de liaison naturel du marqueur de sélection négatif ou un analogue de celui-ci.

5. Procédé selon l'une des revendications 1 à 4 **caractérisé en ce que** l'on utilise une molécule de liaison qui est couplée à une phase solide.

6. Procédé selon la revendication 5 **caractérisé en ce que** l'on utilise des microbilles magnétiques comme phase solide.

7. Procédé selon l'une des revendications 1 à 6 **caractérisé en ce que** l'on utilise une molécule de liaison qui porte un groupe de marquage et/ou un groupe de liaison à une phase solide.

8. Procédé selon la revendication 7 **caractérisé en ce que** le groupe de marquage et/ou le groupe de liaison à une phase solide est choisi parmi la biotine, les dérivés de la biotine, les haptènes, les enzymes et les colorants.

9. Procédé selon la revendication 8 **caractérisé en ce que** l'on utilise la biotine ou un dérivé de la biotine choisi parmi l'iminobiotine, l'aminobiotine et la desthiobiotine.

10. Procédé selon la revendication 8 **caractérisé en ce que** l'on utilise comme enzyme une phosphatase alcaline ou une peroxydase.

11. Procédé selon la revendication 8 **caractérisé en ce que** l'on utilise un colorant fluorescent comme colorant.

12. Procédé selon la revendication 11 **caractérisé en ce que** l'on utilise comme colorant fluorescent la fluorescéine, le phycoérythrine, la rhodamine, la protéine péridinine-chlorophylle ou le rouge Texas.

13. Procédé selon la revendication 9 **caractérisé en ce que** les cellules exprimant le marqueur de sélection négatif sont identifiées par liaison à une phase solide recouverte d'avidine ou de streptavidine.

14. Procédé selon la revendication 10 **caractérisé en ce que** les cellules exprimant le marqueur de sélection négatif sont identifiées par une réaction colorée catalysée par une enzyme.

15. Procédé selon l'une des revendications 11 ou 12 **caractérisé en ce que** les cellules exprimant le marqueur de sélection négatif sont identifiées par analyse par cytométrie de flux.

16. Procédé selon l'une des revendications précédentes **caractérisé en ce que** la cellule est une cellule eucaryote, de préférence une cellule de mammifère et de manière particulièrement préférée une cellule humaine.

17. Vecteur recombiné pour la recombinaison homologue dans des cellules de mammifère comprenant
(a) deux séquences nucléotidiques flanquantes, homologues d'une séquence cible dans une cellule,
(b) une séquence nucléotidique codant un marqueur de sélection positif sous le contrôle d'une séquence de contrôle de l'expression active dans la cellule, qui se trouve à l'intérieur des séquences flanquantes selon (a), et
(c) une séquence nucléotidique codant un marqueur de sélection négatif sous le contrôle d'une séquence de contrôle de l'expression active dans la cellule, qui se trouve à l'extérieur des séquences nucléotidiques homologues flanquantes et dont le produit d'expression est un polypeptide localisé à la surface de la cellule.

18. Vecteur selon la revendication 17 **caractérisé en ce que** les séquences nucléotidiques homologues flanquantes sont choisies dans le domaine d'un gène pour EPO, tPA, G-CSF, GM-CSF, TPO, une interleukine, un interféron, un facteur de croissance, l'insuline ou un facteur de croissance de type insuline.

19. Vecteur selon l'une des revendications 17 ou 18 **caractérisé en ce que** la séquence nucléotidique codant le marqueur de sélection positif est un gène de résistance à la néomycine, la kanamycine, la généticine ou l'hygromycine.

20. Vecteur selon l'une des revendications 17 à 19 **caractérisé en ce que** la séquence nucléotidique codant le marqueur de sélection négatif est une séquence codant un récepteur de LNGF, CD24, LDL, trk ou un fragment membranaire contenant le domaine de liaison de ligand de celui-ci.

21. Vecteur selon l'une des revendications 17 à 20 **caractérisé en ce qu'**il contient en outre une séquence de contrôle de l'expression hétérologue à l'intérieur des séquences flanquantes.

22. Vecteur selon la revendication 21 **caractérisé en ce que** la séquence de contrôle de l'expression comprend un promoteur de CMV.

23. Vecteur selon l'une des revendications 17 à 22 **caractérisé en ce qu'**il contient en outre un gène d'amplification à l'intérieur des séquences flanquantes.

24. Vecteur selon l'une des revendications 17 à 23 **caractérisé en ce que** les séquences nucléotidiques homologues flanquantes comprennent le domaine codant du gène cible ou une partie de celui-ci.

25. Vecteur selon la revendication 24 **caractérisé en ce que** les séquences nucléotidiques homologues flanquantes sont choisies de telle manière que, lors d'une recombinaison homologue, il se produit une mutation dans le domaine codant du polypeptide cible mature.

26. Utilisation d'un vecteur selon l'une des revendications 17 à 25 dans un procédé de recombinaison homologue.
